(19) Europäisches Patentamt
European Patent Office
Office européen des brevets

(11) Veröffentlichungsnummer: **0 228 640**
**A1**

# (12) EUROPÄISCHE PATENTANMELDUNG

(21) Anmeldenummer: 86117362.3

(22) Anmeldetag: 12.12.86

(51) Int. Cl.4: **C07D 403/12** , C07D 209/44 , A61K 31/415

(30) Priorität: 20.12.85 DE 3545206
12.07.86 DE 3623681

(43) Veröffentlichungstag der Anmeldung: 15.07.87 Patentblatt 87/29

(84) Benannte Vertragsstaaten: AT BE CH DE ES FR GB IT LI NL SE

(71) Anmelder: **Beiersdorf Aktiengesellschaft**
**Unnastrasse 48**
**D-2000 Hamburg 20(DE)**

(72) Erfinder: **Cohnen, Erich, Dr.**
**Moorende 74**
**D-2155 Jork(DE)**
Erfinder: **Armah, Ben, Dr.**
**Milcher Strasse 9d**
**D-2000 Hamburg 52(DE)**

(54) **Substituierte 4-Fluor-isoindoline, Verfahren zu ihrer Herstellung und ihre Verwendung sowie diese Verbindungen enthaltende Zubereitungen.**

(57) Substituierte 4-Fluor-isoindoline der allgemeinen Formel I

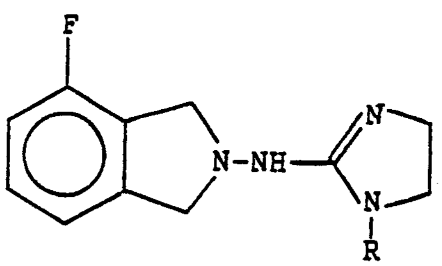

(I)

in der R Wasserstoff oder eine Acylgruppe bedeutet sowie ihre tautomeren Formen und ihre Säureadditionssalze besitzen alpha$_2$-antagonistische Wirkung.

EP 0 228 640 A1

## Substituierte 4-Fluor-isoindoline, Verfahren zu ihrer Herstellung und ihre Verwendung sowie diese Verbindungen enthaltende Zubereitungen

Gegenstand der Erfindung sind neue substituierte 4-Fluor-isoindoline der Formel I

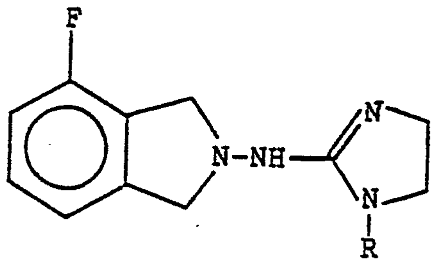

(I)

worin R Wasserstoff oder eine Acylgruppe bedeutet sowie ihre tautomeren Formen und deren Säureadditionssalze sowie Verfahren zu ihrer Herstellung und ihre Verwendung sowie diese Verbindungen enthaltende Zubereitungen.

Der Einfachheit halber sind die erfindungsgemäßen Verbindungen in nur einer durch Formel I wiedergegebenen tautomeren Form definiert. Die Erfindung erstreckt sich jedoch auf alle tautomeren Formen der Verbindungen.

Obgleich pharmazeutisch verträgliche Säureadditionssalze der neuen Verbindungen der Formel I und deren tautomere Formen bevorzugt sind, liegen alle Salze innerhalb des Bereichs der Erfindung. Alle Salze sind wertvoll zur Herstellung der Verbindungen, selbst wenn das spezielle Salz nur als Zwischenprodukt gewünscht wird, wie zum Beispiel, wenn das Salz nur für Zwecke der Reinigung oder Identifizierung gebildet wird, oder wenn es als ein Zwischenprodukt bei der Herstellung eines pharmazeutisch verträglichen Salzes, beispielsweise durch Ionenaustauschverfahrensweisen, verwendet wird.

In den deutschen Offenlegungsschriften 28 16 627 und 29 05 501 sowie der europäischen Patentanmeldung 72 954 ist eine Vielzahl substituierter Isoindoline offenbart. Die erfindungsgemäßen Verbindungen sind dort aber weder beschrieben noch genannt.

Ferner betrifft die Erfindung die neue Verbindung 2-Amino-4-fluoro-isoindolin, ein Verfahren zu ihrer Herstellung sowie ihre Verwendung als Zwischenprodukt für die Herstellung der erfindungsgemäßen Verbindungen der Formel I.

Bevorzugte Acylgruppen sind substituierte und unsubstituierte geradkettige oder verzweigte Alkylcarbonylgruppen bzw. Alkanoylgruppen und substituierte und unsubstituierte Aroylgruppen. Vorzugsweise besitzen alle diese Gruppen jeweils 1 bis 7 Kohlenstoffatome, wobei die Phenylgruppe die bevorzugte Arylgruppe ist. Besonders bevorzugte Alkylcarbonylgruppen sind solche mit 1 bis 4 Kohlenstoffatomen, insbesondere die Formylgruppe, die Acetylgruppe und die Propionylgruppen. Bevorzugter Aroylrest ist der Benzoylrest.

Weiterhin bevorzugte Acylgruppen sind substituierte und unsubstituierte Cycloalkylcarbonylgruppen, vorzugsweise solche mit 4 bis 7 Kohlenstoffatomen. Bevorzugt werden Cyclopropyl-und Cyclohexylringe.

Bevorzugte Substituenten der Acylgruppen, insbesondere der Alkylcarbonylgruppen, sind erstens Alkoxygruppen, vorzugsweise solche mit 1 bis 4 Kohlenstoffatomen, besonders bevorzugt Methoxy-und Ethoxygruppen, zweitens Cycloalkylgruppen mit vorzugsweise 3 bis 6 Kohlenstoffatomen im Ringsystem, vorzugsweise mit Cyclopropyl-und Cyclohexylring und drittens Arylgruppen, insbesondere Phenylgruppen. Besonders bevorzugt werden substituierte Acetylgruppen.

Die folgenden erfindungsgemäßen Verbindungen und deren Säureadditionssalze mit hohem therapeutischen Effekt werden besonders bevorzugt:

4-Fluoro-2-(2-imidazolin-2-ylamino)-isoindolin sowie die folgenden Acylderivate:

4-Fluoro-2-(1-acetyl-2-imidazolin-2-ylamino)-isoindolin
4-Fluoro-2-(1-propionyl-2-imidazolin-2-ylamino)-isoindolin
4-Fluoro-2-(1-methoxyacetyl-2-imidazolin-2-ylamino)-isoindolin

4-Fluoro-2-(1-isobutyroyl-2-imidazolin-2-ylamino)-isoindolin
4-Fluoro-2-(1-cyclopropylcarbonyl-2-imidazolin-2-ylamino)-isoindolin
4-Fluoro-2-(1-phenylacetyl-2-imidazolin-2-ylamino)-isoindolin
4-Fluoro-2-(1-benzoyl-2-imidazolin-2-ylamino)-isoindolin

Von diesen Verbindungen wird 4-Fluoro-2-(2-imidazolin-2-ylamino)-isoindolin mit seinen Säureadditionssalzen besonders bevorzugt. Die Verbindung zeichnet sich durch eine besonders hohe Selektivität und Wirksamkeit aus.

Die erfindungsgemäßen Verbindungen der Formel I und ihre physiologisch verträglichen Säureadditionssalze sin therapeutische Wirkstoffe, besitzen hohe pharmakologische Wirkung und sind wertvolle Arzneimittel. So zeigen sie ein nicht vorhersehbares und therapeutisch wertvolles pharmakologisches Wirkungsspektrum und können aufgrund ihrer selektiven $alpha_2$-antagonstischen Wirkung als Coronartherapeutika dienen. Sie besitzen antikonstriktorische Wirkung an peripheren und koronaren Gefäßen, hemmen ischämiebedingte Arrhythmien, besitzen broncholytische und antiasthmatische Wirkungen, erhöhen den koronaren und peripheren Blutfluß, hemmmen die Thrombozytenaggregation, wirken antihyperglykämisch und vorteilhaft bei metabolischen Störungen, insbesondere solchen des Fettstoffwechsels. Insbesondere können sie durch ihre vasodilatierenden Eigenschaften als Antihypertonika bei pulmonaler oder arterieller Hypertonie Anwendung finden. Ferner eignen sich die erfindüngsgemäßen Substanzen zur Behandlung von Glaukom, koronaren und peripheren Durchblutungsstörungen, Migräne, Angina pectoris, Myokardinfarkt, Morbus Raynaud, Asthma und metabolischen Störungen, Diabetes, Fettsucht sowie Lungen-, Bronchial-und Coronarerkrankungen und ischämiebedingten Arrhythmien. Die Verbindungen der Formel I und ihre Salze besitzen ebenfalls Blutplättchen-aggregationshemmende Eigenschaften. Sie können zur Behandlung thromboembolischer Erkrankungen dienen.

Die Verbindungen der vorliegenden Erfindung können oral oder parenteral angewendet werden Die Einzeldosis beträgt beim Menschen 1 bis 50 mg, vorzugsweise 1 bis 5 mg. Diese Dosierung wird bevorzugt für die Behandlung von Hypertonie.

Die Tagesdosis ist, wie für Alpha-Antagonisten üblich, individuell abzustimmen, weil sie von der Rezeptorenempfindlichkeit und dem Sympathikotonus des Patienten abhängt. Zweckmäßigerweise wird die Behandlung mit niederen Dosen begonnen und dann gesteigert.

Zur Behandlung des erhöhten Augeninnendrucks werden vorzugsweise 0,01 bis 20 %ige isotone Lösungen in Dosierungen von einem oder mehreren Tropfen ein oder mehrmals täglich lokal am Auge angewendet.

Gemäß der Erfindung werden pharmazeutische Zusammensetzungen geschaffen, die eine Verbindung der Formel I oder deren pharmazeutisch verträglichen Salze, zusammen mit einem pharmazeutisch verträglichen Verdünnungsmittel oder Träger enthalten.

Die Verbindungen gemäß der Erfindung können allein oder mit üblichen pharmazeutisch verträglichen Verdünnungsmitteln oder Trägern und gegebenenfalls mit anderen Hilfsmitteln vermischt und beispielsweise oral oder parenteral verabreicht werden. Sie können oral in Form von Tabletten, Dragees, Zäpfchen, Pulvern, Sirups, Suspensionen und Flüssigkeiten oder parenteral in Form von Lösungen oder Suspensionen verabreicht werden. Oral zu verabreichende Präparate können ein oder mehrere Zusätze, wie Süßungsmittel, Aromatisierungsmittel, Farbstoffe und Konservierungsmittel, enthalten. Tabletten können den Wirkstoff mit üblichen, pharmazeutisch verträglichen Hilfsmitteln vermischt enthalten, zum Beispiel inerten Verdünnungsmitteln wie Calciumcarbonat, Natriumcarbonat, Lactose und Talk, Granulierungsmitteln und Mitteln, die den Zerfall der Tabletten bei oraler Verabreichung fördern, wie Stärke oder Alginsäure, Bindemitteln wie Stärke oder Gelatine, Gleitmitteln wie Magnesiumstearat, Stearinsäure und Talk.

Geeignete Trägerstoffe sind beispielsweise Milchzucker (Lactose), Gelatine, Maisstärke, Stearinsäure, Äthanol, Propylenglycol, Äther des Tetrahydrofurylalkohols und Wasser.

Die Tabletten können nach bekannten Arbeitsweisen überzogen werden, um den Zerfall und die Resorption im Magen-Darmtrakt zu verzögern, wodurch die Aktivität des Wirkstoffs sich über eine längere Zeitspanne erstrecken kann. Ebenso kann in den Suspensionen der Wirkstoff mit Hilfsmitteln vermischt sein, die für die Herstellung solcher Zusammensetzungen üblich sind, zum Beispiel Suspendiermitteln wie Methylcellulose, Tragacanth oder Natriumalginat, Netzmitteln wie Lecithin, Polyäthylenstearat und Polyoxyäthylensorbitanmonooleat, und Konservierungsmitteln wie Äthylparahydroxybenzoat. Kapseln können den Wirkstoff als einzigen Bestandteil oder vermischt mit einem festen Verdünnungsmittel wie Calciumcarbonat, Calciumphosphat oder Kaolin enthalten. Die injizierbaren Präparate werden ebenfalls in an sich bekannter Weise formuliert. Die pharmazeutischen Präparate können den Wirkstoff in einer Menge von 0,1 bis 90%, insbesondere 1 bis 90%, enthalten, wobei der Rest ein Trägerstoff oder Zusatzstoff ist. Im Hinblick auf die Herstellung und Verabreichung werden feste Präparate, wie Tabletten und Kapseln, bevorzugt. Vorzugsweise enthalten die Präparate den Wirkstoff in einer Menge von 2 mg.

Aufgabe der Erfindung war es, unter Berücksichtigung der Vielzahl ähnlich strukturierter Verbindungen, die erfindungsgemäßen, überraschend selektiv und hochwirksamen Verbindungen aufzufinden.

Die erfindungsgemäßen Verbindungen, nämlich die Verbindung 4-Fluoro-2-(2-imidazolin-2-ylamino)-isoindolin und deren Acylderivate, besitzen diese hervorragenden Eigenschaften gegenüber anderen, in der Struktur ähnlichen Verbindungen in nicht vorhersehbarer Weise. Man nimmt an, daß diese besonderen, spezifischen Wirkungen von der speziellen Struktur der erfindungsgemäßen Verbindungen abhängen.

Es wird angenommen, daß diese überraschende überlegene Wirksamkeit der neuen Verbindungen auf der 4-Fluor-Substitution des 2-(2-Imidazolin-2-ylamino)-isoindolins beruht.

Die neuen Verbindungen der Formel I können nach folgenden Verfahren hergestellt werden:

a) Durch Umsetzung von 2-Amino-4-fluoro-isoindolin mit einer Verbindung der Formel II

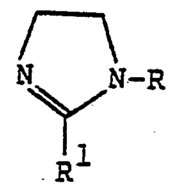

(II)

in der R' eine nucleophil austauschbare Gruppe wie die Alkylthiogruppe oder ein Halogenatom und R ein Wasserstoffatom oder eine Acylgruppe wie oben angegeben bedeuten. Bevorzugte Acylgruppen sind Acetyl-, Propionyl-oder Butyrylgruppen. Bevorzugt wird die Acetylgruppe. Die Umsetzung wird in einem Alkohol, zweckmäßigerweise n-Amylalkohol, als Lösungsmittel bei Siedetemperatur durchgeführt.

Besonders geeignet für diese Reaktion sind Methylthioverbindungen, z.B. in Form ihrer Hydrojodide.

Zur Herstellung der erfindungsgemäßen Verbindung der Formel I, in der R Wasserstoff bedeutet, lassen sich diese Acylgruppen mit verdünnten Säuren bei Raumtemperatur abspalten.

b) Durch Umsetzung von 2-Amino-4-fluoro-isoindolin mit einer Verbindung der allgemeinen Formel III

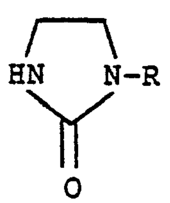

(III)

in der R eine Acylgruppe mit der angegebenen Bedeutung ist.

Man führt die Umsetzung beispielsweise mit einem 1-Acyl-imidazolidin-2-on in Gegenwart von mindestens 2 mol Phosphoroxitrichlorid bei Temperaturen von 50 -100°C bei einer Reaktionsdauer von 70 bis 4 Stunden aus.

Bevorzugte Acylgruppen besitzen 2 bis 4 Kohlenstoffatome.

Nach der Kondensation läßt sich die Acylgruppe mit Säuren oder Basen hydrolytisch, vorzugsweise durch Erhitzen in Äthanol unter sauren Bedingungen, abspalten.

Mit Acylierungsmitteln, wie Acylhalogeniden, in denen die Acylgruppe die obengenannte Bedeutung hat, lassen sich aus dem erhaltenen 4-Fluoro-2-(2-imidazolin-2-ylamino)-isoindolin die erfindungsgemäßen Acylderivate ebenfalls erhalten.

Die neue Verbindung 2-Amino-4-fluoro-isoindolin wird nach dem folgenden Verfahren erhalten:

Aus 4-Fluor-phthalsäureanhydrid und tert.-Butylcarbazat erhält man durch Erhitzen in einem geeigneten Lösungsmittel das Phthalimid der Formel (IV)

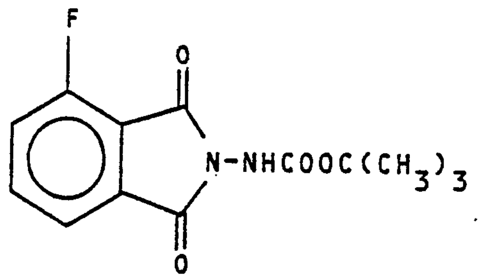

(IV)

das durch Reduktion mit Lithium-aluminiumhydrid in das Isoindolin der Formel (V) überführt wird

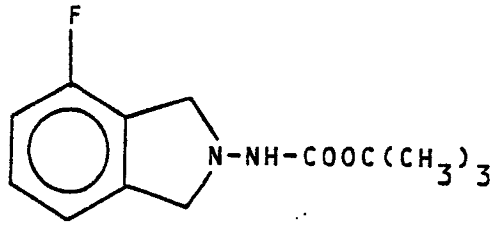

(V)

Durch Hydrolyse mit Mineralsäuren wird die tert.-Butoxycarbonylgruppe entfernt und man erhält 2-Amino-4-Fluoro-isoindolin.

Die Ausgangsverbindungen der Formeln II und III sind bekannt oder können nach bekannten Verfahren erhalten werden.

Die Verbindungen der allgemeinen Formel I können entweder als Basen oder in Form ihrer Salze aus den Reaktionsgemischen isoliert werden. Sie lassen sich als Basen mit geeigneten anorganischen oder organischen Säuren nach bekannten Verfahren in die Salze überführen.

Bevorzugt werden physiologisch verträgliche Salze der Verbindungen der Formel I. Hierfür sind als anorganische Säuren beispielsweise Halogenwasserstoffsäuren, zum Beispiel Salzsäure oder Schwefelsäure und als organische Säuren zum Beispiel Fumarsäure, Maleinsäure, Zitronensäure und Weinsäure geeignet. Zur Herstellung wird die heiße alkoholische Lösung der Base mit der alkoholischen Lösung einer geeigneten Säure versetzt und man erhält nach Etherzusatz das Salz.

Besonders bevorzugte Salze sind: Hydrochloride, Hydrogensulfate, Hydrogenphosphate, Fumarate, Maleinate, Lactate und Citrate.

Die folgenden Beispiele dienen zur Erläuterung der Erfindung:

Beispiel 1

4-Fluoro-2-(2-imidazolin-2-ylamino)-isoindolin

6,0 g (0,03 mol) 2-Amino-4-fluoro-isoindolin-hydrochlorid und 4,5 g (0,035 mol) 1-Acetyl-imidazolidin-2-on werden in 70 ml Phosphoroxitrichlorid 4 Stunden auf 100°C erwärmt. Nach Entfernen des $POCl_3$ im Vakuum wird der Rückstand in 100 ml Ethanol gelöst und 3 Stunden zum Sieden erhitzt. Das Lösungsmittel wird abdestilliert, der Rückstand mit 5 N NaOH versetzt und die Rohbase mit Methylenchlorid extrahiert. Durch Umkristallisation aus Toluol erhält man 5,9 g 4-Fluoro-2-(2-imidazolin-2-ylamino)-isoindolin.

Fp. 168-170°C.

Beispiel 2

4-Fluoro-2-(2-imidazolin-2-ylamino)-isoindolin-maleinat

Man löst 2,0 g der Base (Beispiel 1) in 100 ml heißem Essigester und versetzt mit einer heißen Lösung von 1 g Maleinsäure in Essigester.
Ausbeute: 2,6 g
Fp. 189-191 °C.

Beispiel 3

2-Amino-4-fluoro-isoindolin

a) 11,6 g (0,07 mol) 3-Fluoro-phthalsäure-anhydrid werden in 200 ml o-Xylol suspendiert und portionsweise mit 9,3 g (0,07 mol) Carbazinsäure-tert.-butylester versetzt. Man erhitzt 2 Stunden unter Abdestillation von Wasser. Nach Abkühlen kristallisieren 17,1 g 3-Fluoro-N-(tert.-butyloxycarbonyl)-amino-phthalimid aus.
Fp. 183-184°C.

b) 5,6 g $LiAlH_4$ werden in 70 ml absolutem Tetrahydrofuran suspendiert. Bei 30-45°C wird eine Lösung von 15,9 g (0,06 mol) des Phthalimids aus Stufe a) in 150 ml THF unter $N_2$ zugetropft. Man rührt noch 3 Stunden bei Raumtemperatur und tropft dann 20 ml 2 N NaOH zu. Der Niederschlag wird abfiltriert und das Lösungsmittel im Vakuum abgezogen. Der Rückstand wird mit Toluol heiß extrahiert. Nach Abdampfen des Toluols erhält man 8,6 g 4-Fluoro-N-tert.-butyloxycarbonylamino-isoindolin als kristalline Substanz, die aus Cyclohexan umkristallisiert wird.
Fp. 106-108°C.

c) 3,8 g 4-Fluoro-N-tert.-butyloxycarbonyl-amino-isoindolin werden bei Raumtemperatur in 30 ml konzentrierte Salzsäure eingetragen. Man rührt 1 Stunde bei Raumtemperatur nach und destilliert danach das Lösungsmittel im Vakuum ab. Der Rückstand wird mit heißem i-Propanol behandelt; nach Abkühlen auf 10°C wird der Niederschlag abgesaugt. Man erhält 2,1 g 2-Amino-4-fluoro-isoindolin-hydrochlorid.
Fp. 208-210°C (Z.).

Beispiel 4

4-Fluoro-2-(1-acetyl-2-imidazolin-2-ylamino)-isoindolin

33,2 g (0,18 mol) 2-Amino-4-fluoro-isoindolin-hydrochlorid und 24,8 g (0,19 mol) 1-Acetyl-imidazolidin-2-on werden in 70 ml Phosphoroxitrichlorid 4 Stunden auf 160°C erwärmt. Das überschüssige $POCl_3$ wird im Vakuum entfernt, der Rückstand auf Eis gegossen und mit 10%iger NaOH alkalisch gestellt. Die Acetylverbindung wird mit $CH_2Cl_2$ extrahiert und die Methylenchloridphase mehrmals mit Wasser gewaschen. Nach Entfernen des Lösungsmittels erhält man 30,5 g 4-Fluoro-2-(1-acetyl-2-imidazolin-2-ylamino)-isoindolin.
Fp. 121-123°C (Toluol)

Beispiel 5

Herstellung von Tabletten

Tabletten, welche die nachstehend angegebenen Bestandteile enthalten, werden nach bekannten Arbeitsweisen hergestellt. Diese sind für die Behandlung von den vorstehend genannten Krankheiten in einer Dosierungsmenge von 1 -2 Tabletten einmal bis zweimal täglich geeignet.

| | Tablette A | Tablette B |
|---|---|---|
| 4-Fluoro-2-(2-imidazolin-2-ylamino)-isoindolin-maleinat | 2 mg | 5 mg |
| Lactose | 90 mg | 90 mg |
| Maisstärke | 5 mg | 5 mg |
| Magnesiumstearat | 1 mg | 1 mg |

Beispiel 6

Herstellung von Ampullen

Ampullen, die die im folgenden genannten Bestandteile enthalten, lassen sich in bekannter Weise herstellen. Wirkstoff und Natriumchlorid werden in Wasser gelöst und unter Stickstoff in Glasampullen abgefüllt. Diese sind für die Behandlung von den vorstehend genannten Krankheiten in einer Dosierungsmenge von 0,5mg einmal bis zweimal täglich geeignet.
4-Fluoro-2-(2-imidazolin-2-ylamino)-isoindolin-maleinat 0,5 mg
Natriumchlorid 18 mg
dest. Wasser ad 2,0 g

Beispiel 7

Herstellung von Augentropfen

Augentropfen, die die folgenden Bestandteile enthalten, lassen sich in bekannter Weise herstellen. Sie können in einer Dosis von ein bis zwei Tropfen pro Auge ein bis zweimal täglich, vorzugsweise ein Tropfen zweimal täglich, verwendet werden:
4-Fluoro-2-(2-imidazolin-2-ylamino)-isoindolin-maleinat/ 0,5 g
isoindolin-maleinat/ 0,5 g
isotone wäßrige Lösung (Ringerlösung)/ ad 100 ml

**Ansprüche**

1. Substituierte 4-Fluor-isoindoline der allgemeinen Formel I

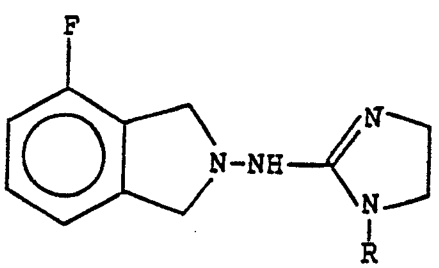

(I)

in der R Wasserstoff oder eine Acylgruppe bedeutet sowie ihre tautomeren Formen und ihre Säureadditionssalze.

2. 4-Fluoro-2-(2-imidazolin-2-ylamino)-isoindolin.

3. 4-Fluoro-2-(1-acetyl-2-imidazolin-2-ylamino)-isoindolin.

4. Verbindungen der Formel 1 gemäß Anspruch 1, dadurch gekennzeichnet, daß die Acylgruppen R substituierte und unsubstituierte Alkylcarbonyl-, Cycloalkylcarbonyl und Aroylgruppen sind.

5. 2-Amino-4-fluoro-isoindolin.

6. Verfahren zur Herstellung der Verbindungen der Formel I gemäß Anspruch 1, dadurch gekennzeichnet, daß man 2-Amino-4-fluoro-isoindolin mit einer Verbindung der Formel II

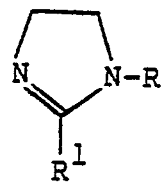

(II)

in der R die oben angegebene Bedeutung hat und R' eine nucleophil austauschbare Gruppe ist, umsetzt und zur Herstellung der Verbindungen in denen R Wasserstoff bedeutet die Acylgruppe abspaltet.

7. Verfahren zur Herstellung der Verbindungen der Formel I gemäß Anspruch 1, dadurch gekennzeichnet, daß man 2-Amino-4-fluoro-isoindolin mit einer Verbindung der Formel III

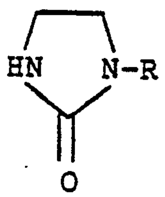

(III)

in der R die oben angegebene Bedeutung hat, umsetzt und zur Herstellung der Verbindungen in denen R Wasserstoff bedeutet die Acylgruppe abspaltet.

8. Verfahren zur Herstellung der Acylverbindungen der Formel I gemäß Anspruch 1, dadurch gekennzeichnet, daß man 4-Fluoro-2-(2-imidazolin-2-ylamino)-isoindolin mit Acylreste R enthaltenden Acylierungsmitteln umsetzt.

9. Verfahren zur Herstellung von 2-Amino-4-fluoro-isoindolin, dadurch gekennzeichnet, daß man 3-Fluorphthalsäureanhydrid mit tert.-Butylcarbazat umsetzt, das erhaltene substituierte Phthalimid durch Reduktion in das entsprechende substituierte Isoindolin überführt und aus diesem durch Abspalten des tert.-Butoxycarbonylrestes 2-Amino-4-fluor-isoindolin erhält.

10. Pharmazeutisches Präparat, dadurch gekennzeichnet, daß es eine oder mehrere der Verbindungen gemäß Anspruch 1 oder deren physiologisch verträgliche Salze und gegebenenfalls übliche Trägerstoffe und/oder Verdünnungsmittel enthält.

11. Verbindungen der Formel I gemäß Anspruch 1 zur Anwendung als therapeutische Wirkstoffe.

Europäisches Patentamt

# EUROPÄISCHER RECHERCHENBERICHT

Nummer der Anmeldung

EP 86117362.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X,D | DE - A1 - 2 905 501 (BEIERSDORF)<br>* Ansprüche 1,2,3,7; Beispiel 1 *<br>-- | 1,6,7,<br>10,11 | C 07 D 403/12<br>C 07 D 209/44<br>A 61 K 31/415 |
| D,A | DE - A1 - 2 816 627 (BEIERSDORF)<br>* Formel I, Anspruch 3 *<br>-- | 1,10,<br>11 | |
| A | CHEMICAL ABSTRACTS, Band 99, Nr. 13, 26. September 1983, Columbus, Ohio, USA<br>SCHLICKER, E.; GOETHERT, M.; KOESTERMANN, F.; CLAUSING, R. "Effects of α-adrenoceptor antagonists on the release of serotonin and noradrenaline from rat brain cortex slices. Influence of noradrenaline uptake inhibition and determination of $pA_2$ values"<br>Seite 64, Spalte 1, Zusammenfassung-Nr. 99 175q<br>& Naunyn-Schmiedeberg's Arch. Pharmacol. 1983, 323(2), 106-13<br>---- | 10 | RECHERCHIERTE SACHGEBIETE (Int. Cl.4)<br>C 07 D 403/00<br>C 07 D 209/00 |

Der vorliegende Recherchenbericht wurde für alle Patentansprüche erstellt.

| Recherchenort | Abschlußdatum der Recherche | Prüfer |
|---|---|---|
| WIEN | 05-03-1987 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82